(19) Europäisches Patentamt / European Patent Office / Office européen des brevets

(11) **EP 3 913 369 A1**

(12) **EUROPEAN PATENT APPLICATION**

(43) Date of publication:
24.11.2021 Bulletin 2021/47

(51) Int Cl.:
*G01N 33/569* (2006.01)

(21) Application number: 20175656.6

(22) Date of filing: 20.05.2020

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Diesse Diagnostica Senese S.p.a.**
**53035 Monteriggioni, Siena (IT)**

(72) Inventors:
• **BROGI, ALESSANDRA**
**53035 Monteriggioni (IT)**
• **RICCI, VERONICA**
**53035 Monteriggioni (IT)**
• **VERDIANI, SILVANA**
**53035 Monteriggioni (IT)**

(74) Representative: **Capasso, Olga**
**De Simone & Partners S.r.l.**
**Via Vincenzo Bellini, 20**
**00198 Roma (IT)**

(54) **METHOD FOR INACTIVATING SARS-COV-2 AND USES THEREOF**

(57) **Field of invention**

The invention relates to an inactivation method of an inactivated SARS-CoV-2 virus, to be used in the preparation of immunological assays for the detection of antibodies in samples.

**EP 3 913 369 A1**

**Description**

**Field of invention**

The invention relates to an inactivation method of an inactivated SARS-CoV-2 virus, to be used in the preparation of immunological assays for the detection of antibodies in samples.

**State of the Art**

A novel human coronavirus that is named severe acute respiratory syndrome coronavirus 2 (SARS-CoV-2, named also as COVID-19 syndrome) emerged in Wuhan, China, in late 2019. The associated disease COVID-19 turned into a global humanitarian crisis and is causing 2.319.066 confirmed cases with 157.970 confirmed deaths (7%) [1]. COVID-19 is a new syndrome, distinct from other diseases caused by coronaviruses, such as Severe Acute Respiratory Syndrome (SARS) and Middle East Respiratory Syndrome (MERS) [2].

Diagnostic assays for the detection of SARS-CoV-2, as stressed by World Health Organization (WHO), are important to fast and effectively find all suspected cases of COVID-19 in order to isolate and treat confirmed cases but, moreover, for tracing their contacts and avoid the virus to spread.

Diagnostic tests to confirm cases of COVID-19 are based on detection of unique sequences of virus RNA by nucleic acid amplification tests such as real-time reverse-transcription polymerase chain reaction (rRT-PCR) with confirmation by nucleic acid sequencing when necessary [3]. Serological screening and test can aid investigation of an ongoing outbreak and retrospective assessment of the attack rate or extent of an outbreak. In case of negative PCR and epidemiological link to COVID-19 infection, serology tests could support diagnosis. In addition, antibody testing could be used to verify the efficacy of a vaccine or to detect asymptomatic cases. In fact, antibodies reveal evidence of a previous or on-going infection [4].

The development of some serological immunoassays has been reported for the detection of SARS-CoV-2 viral proteins and antibodies in the serum or plasma [5]. The most widely used biomarkers for the detection of SARS-CoV-2 infection are IgM and IgG antibodies produced starting from the second week of viral infection. The IgM response occurs earlier than that of IgG, but it then decreases and disappears. On the other hand, IgGs can persist after infection for a long time and may have a protective role.

The strategy for the development of the serological immunoassay can be based on the use of individual viral antigens or on the combined use of different viral antigens. Different immunoassays for the determination of antibody levels in COVID-19 samples have been developed using as antigens SARS-CoV-2 proteins or recombinant forms of these.

SARS-CoV-2 structural proteins, used as immunoassay antigens, most often include spike (S), and nucleocapsid (N) proteins. The S protein, located on the surface of the virus, is important for the attachment to host cells and has been reported to be highly immunogenic. The N protein is involved in the transcription and replication of viral RNA, and interference with cell cycle processes of host cells. In many coronaviruses, the N protein has high immunogenic activity and is abundantly expressed during infection. The sensitivity of ELISA based on recombinant S protein for IgM detection is higher than that developed with N protein [6]. An IgA response may appear and grow early than IgM, leading to a stronger and more persistent response than IgM [7].

The specificity of the serological tests strongly depends on the type and quality of the used proteins, this because of cross-reactivity with other coronaviruses can lead to false positives results. In fact, for example, S protein of SARS-CoV-2 shares a 75% of amino acid identity with that of SARS, and about 50-60% with other common cold-causing coronaviruses [4].

In addition, it was shown that conformational changes occurring in the S protein of SARS-CoV-2 are fundamental for membrane fusion and viral entry [8,9]. Therefore, in case of conformational epitopes, the use of recombinant proteins may not be effective for the antibody detection. The development of a diagnostic immunoassay based on the use of the inactivated viral antigen SARS-CoV-2 could be a solution and it is, thus, the object of this invention.

Moreover, this strategy allows the development of a diagnostic immunoassay that can be easily related to the immunofluorescent technique, which is a reference method for virology.

**Summary of the invention**

The present invention relates to the preparation of an inactivated purified whole virus SARS-CoV-2 at large scale and its use for the production of a diagnostic immunoassay for detection of antibodies of class IgG and/or IgM and/or IgA associated to COVID-19 in biological samples, preferably human samples.

In the present invention it was surprisingly found that inactivated purified whole virus SARS-CoV-2, in particular of the strain 2019-nCoV/Italy-INMI1 (GenBank: SARS-CoV-2/INMI1-Isolate/2020/Italy: MT066156), has high selectivity and specificity for antibodies associated to COVID-19.

In particular the present invention is advantageous in that it is shown that:

i) antibodies specific to the inactivated purified whole virus SARS-CoV-2 are present in serum samples after disease recovery and in patients but are not present in negative patients;

ii) all of 3 different types of antibodies, namely IgG, IgM, and IgA, can be detected in serum samples using the inactivated purified whole virus SARS-CoV-2 of the invention;

iii) given that IgG, IgM, and IgA are directed against different viral proteins, the use of the inactivated purified whole virus SARS-CoV-2 instead of single or selected recombinant viral proteins provides a more sensitive diagnostic immunoassay;

iv) the different reactivity of IgG in reducing and non-reducing conditions suggest that the reduction of the disulfide bonds of the viral proteins may alter their antigenicity.

Therefore, the invention provides a method to inactivate SARS-CoV-2 whole virus particles from an isolated biological sample, comprising the steps of:

a) inactivating SARS-CoV-2 whole virus particles by at least two additions of an inactivating reagent to said sample and optionally

c) purifying inactivated SARS-CoV-2 whole virus particles.

Preferably the additions of 0,1% (v/v) of β-propiolactone are performed at temperature ranging from 15°C to 37°C for at least 1 hour, preferably for 3 hours.

Preferably the SARS-CoV-2 whole virus particles belong to the strain 2019-nCoV/Italy-INMI1 (GenBank: SARS-CoV-2/INMI1-Isolate/2020/Italy: MT066156), or to a natural or recombinant derivative thereof.

The invention also provides a method of detecting one or more anti-SARS-CoV-2 virus specific antibody in a biological sample, the method comprising:

a) incubating the biological sample with inactivated purified SARS-CoV-2 whole virus particles as prepared as described above under suitable conditions to obtain a SARS-CoV-2 virus/antibody complex;

b) optionally washing to remove unbound material;

c) detecting said SARS-CoV-2 virus /antibody complex.

Preferably the anti-SARS-CoV-2 virus antibody is an IgG, IgM and/or an IgA antibody.

Preferably said inactivated purified SARS-CoV-2 whole virus particles are pre-immobilized to a solid support.

Preferably the solid support is a chip, column matrix material, a culture plate, a tube, a dish, a flask, a microtiter plate, a bead, microsphere, reactor vessel, wells, polystyrene or a combination thereof, preferably the solid support is a paramagnetic particle (PMP) or a latex magnetic particle (LMP).

Preferably the detecting step is performed by means of a labelled secondary antibody, preferably a monoclonal antibody.

Preferably the secondary antibody is labeled with a radioactive isotope, or an enzyme, preferably the enzyme is peroxidase, alkaline phosphatase, β Galactosidase or acetylcholinesterase.

Preferably the detecting step comprises measuring a signal from the label and comparing the signal to a control signal from a control biological sample known to be negative for SARS-CoV-2 virus antibodies.

Preferably said inactivated purified SARS-CoV-2 whole virus particles comprise SARS-CoV-2 at least one of the following proteins :virus spike glycoprotein, nucleocapsid protein, membrane protein, an envelope protein or an immunogenic fragment thereof.

Preferably the biological sample is blood, serum, plasma, body fluid, saliva and other secretions from the subject or tissue or cell extracts.

Preferably the biological sample is obtained from the subject at least 3 days for IgM and IgA and at least 10 days for IgG after onset of symptoms of SARS-CoV-2 virus infection or is at risk of exposure to SARS-CoV-2 virus, or the subject has recovered from SARS-CoV-2 virus infection. Preferably the method further comprises a quantification of amount of specific SARS-CoV-2 virus antibodies in the biological sample.

Preferably the amount of the SARS-CoV-2 virus antibody in the biological sample is proportional to the amount of the complex detected.

The invention further provides a kit for working the method as defined above comprising:

a) a solid support coated with inactivated purified SARS-CoV-2 whole virus particles,

b) at least one labeled secondary antibody able to bind to IgG or IgM or IgA,

c) a negative control;

d) a positive control;
e) a cut-off control or calibrator;
f) a sample diluent;
g) a substrate solution;
h) a washing solution;
i) optionally a stop solution.

The invention also provides the use of inactivated purified SARS-CoV-2 whole virus particles obtained by the method as defined above for detecting SARS-CoV-2 antibodies wherein said SARS-CoV-2 virus is strain 2019-nCoV/Italy-INMI1 (GenBank: SARS-CoV-2/INMI1-Isolate/2020/Italy: MT066156) or natural or recombinant derivative thereof.

In the present invention the method for the production of the inactivated purified whole virus SARS-CoV-2 may comprise: virus propagation in cell cultures, inactivation, and purification.

In particular, SARS-CoV-2 whole virus may be propagated in cells such as in epithelial cells (e.g. VERO cell lines and subclones), inactivation and purification, in particular by sedimentation the inactivated SARS-CoV-2 whole virus particles to recover the whole virus particles containing the proteins.

In the present invention the biological sample may be obtained from the subject at least 3, 4, 5 or 6 days for IgM and IgA and at least 10, 11, 12, 13, 14, 15, 16, 17 or 18 days for IgG after onset of symptoms of SARS-CoV-2 virus infection.

The immunoenzymatic method may comprises the following steps:

1) coating of the inactivated purified whole virus SARS-CoV-2 to a solid support (e.g. chips, microspheres, polystyrene, reactor vessels or wells, microtiter plate);
2) addition of the human sample to be tested to the coated solid support and incubation under conditions suitable for the formation of the antigen/antibody immune binding complex;
3) washing to remove any unbound material;
4) addition of the conjugated secondary antibodies against human antibodies and incubation under conditions suitable for the formation of antigen/antibody immune complexes;
5) washing to remove any unbound material;
6) detection of the presence of antigen/antibody immune binding complexes.

The invention will be illustrated by means of non-limiting examples in reference to the following figures.

Figure 1. Gel electrophoresis performed in non-reducing conditions with a 10% separation. Detection by silver staining. On the right of the molecular-weight size marker, are reported four different production batches of the inactivated purified whole virus SARS-CoV-2 (i.e. lanes A, B, C, and D). The predominant band is albumin resulting from cell culture medium.

Figure 2. Gel electrophoresis performed in reducing (left of size marker), and non-reducing (right of size marker) conditions. Detection by silver staining. The gel was performed at different concentrations of the purified inactivated viral antigen SARS-CoV-2 (2 and 5 $\mu$l).

Figure 3. Western Blot performed in reducing conditions and detected with five COVID-19 positive serum samples (lanes 1-5) and with six COVID-19 negative positive serum samples (lanes 6-11). Detection is performed with an anti-human IgG.

Figure 4. Western Blot performed in non-reducing conditions and detected with one COVID-19 positive serum sample from a recovered patient. Detection is performed with anti-human IgG, IgA, and IgM.

## Detailed description of the invention

## Methods

## Virus propagation

VERO E6 cells (ATCC, CRL-1586) (cultivated and collected) are propagated by cultured with conventional techniques [10]. The cell substrates are infected with SARS-CoV-2 virus (strain 2019-nCoV/Italy-INMI1, the complete sequence was submitted to GenBank (SARS-CoV-2/INMI1-Isolate/2020/Italy: MT066156) and is available on GISAID website (BetaCoV/Italy/INMI1-isl/2020: EPI_ISL_410545) at specific multiplicity of infection MOI value of 0,05 and are incubated at 37°C for 46-50 hours in roller bottles (850 cm$^2$ each). After incubation, the cytopathic effect (cpe) distinctive for SARS-CoV-2 is evaluated observing by microscopy on the VERO E6 cell monolayer the cell rounding, detachment, and degeneration [11]. The virus titer is quantified by using the Reed-Muench method and calculating the TCID50/ml [12]. After 48 hours the cpe is completed and viruses are harvested and frozen at - 80°C. Samples are taken to evaluate the infectious titer of the virus.

**Virus inactivation**

After thawing, the viruses are inactivated with β-propiolactone (Natalex, CAS 57-57-8). To ensure inactivation, at least two consecutive additions of 0,1% [v/v] of β-propiolactone are added to the fixed volume of the viral suspension. Each cycle of β-propiolactone treatment is incubated at room temperature for 3 hours. Then, the viral suspension is incubated for 2 hours at 37°C. The inactivation control is checked by verifying the disappearance of live viruses through cpe on a sub-culture of inactivated material. This control is performed using live viruses with a $10^4$-$10^5$ TCID50/ml as reference. By microscopy the presence/absence of cpe is observed and the TCID50/ml titer is calculated. The virus is inactivated, if the VERO E6 cell monolayer does not show the SARS-CoV-2 cpe and the TCID50/ml is <$1*10^{1,5}$. In case of incomplete inactivation, the treatment with 0,1% of β-propiolactone is repeated and checked. The inactivated viruses are frozen at -80°C.

**Purification**

Purification of the inactivated viruses is performed first by clarification by centrifugation at 10.000 g to remove cell debris. The supernatant is collected and concentrated by ultracentrifugation at 100.000 g. The inactivated viruses are present in the pellet, the supernatant is collected and the pellet is sonicated and resuspended in PBS (pH 7,2-7,8). The inactivated purified viruses are distributed in vials and frozen at -80°C.

**Immunoenzymatic method**

The present invention immunoenzymatic method for the diagnosis of COVID-19 in a subject comprises the step of detecting antibodies specific for SARS-CoV-2 in a human sample by reacting under proper conditions said human sample with the inactivated purified whole virus SARS-CoV-2 of the invention to produce an immunocomplex, which is then detected by a conjugated secondary antibody.

The immunoenzymatic method comprises the following steps:

1) coating of the inactivated purified whole virus SARS-CoV-2 to a solid support (e.g. chips, microspheres, polystyrene, reactor vessels or wells, microtiter plate);
2) addition of the human sample to be tested to the coated solid support and incubation under conditions suitable for the formation of the antigen/antibody immune binding complex;
3) washing to remove any unbound material;
4) addition of the conjugated secondary antibodies against human antibodies and incubation under conditions suitable for the formation of antigen/antibody immune complexes;
5) washing to remove any unbound material;
6) detection of the presence of antigen/antibody immune binding complexes wherein the antibody is specific for SARS-CoV-2.

The inactivated purified whole virus SARS-CoV-2 (1:100) is coated to the microtiter plates in citrate buffer (pH 2,9-3,1) by incubating at room temperature for 24 hours. After incubation, the plates are washed, treated with a saturating agent (e.g. buffered solution of PBS at 7,2-7,4 pH containing 6-7% sucrose and 0,5-1,0% BSA), and then dried. The human sample diluted 1:100 is applied to the microtiter plate and is incubated at room temperature for 1 hour. After incubation and washings, the conjugated secondary antibody is added to the microtiter plate and incubated at room temperature for 1 hour. After incubation and washings, detection is performed depending on the used immunoenzymatic technique (e.g. addition of substrate and stop solution to read at 405-450 nm or 620 nm in case of ELISA). The results of the test are calculated by dividing the absorbance of each sample by the absorbance of the cut-off value (i.e. OD mean of negative sera plus two standard deviations). Results are expressed as index (OD sample / OD cutoff). Results are positive if the index is above 1,1.

The secondary antibody may be an anti-human-IgG or/ and-IgM or/ and-IgA antibody. Different secondary antibodies can be used to detect the presence of specific antibodies in the sample and the relative amounts of different antibodies.

The diagnostic kits for the detection of antibodies specific for SARS-CoV-2 may be based on an immunoenzymatic assay comprising the following reagents:

- a solid support, such as a microtiter plate, coated with the inactivated purified whole virus SARS-CoV-2;

- a labelled anti-human antibody, for instance an enzyme-conjugated anti-human antibody (e.g. such as any monoclonal or polyclonal antibodies anti-human or other species IgG, IgM, and/or IgA labeled with horseradish peroxidase in 2% newborn calf serum in PBS solution);
- a negative control (for instance 1% BSA in PBS solution containing 0,2% Tween-20);
- a positive control (for instance specific anti-SARS-CoV-2 monoclonal/polyclonal antibodies in PBS solution in containing 1% BSA and 0,2% Tween-20);
- a cut-off control or calibrator (for instance specific anti-SARS-CoV-2 monoclonal/polyclonal antibodies in PBS solution in containing 1% BSA and 0,2% Tween-20);
- a sample diluent (for instance 2% newborn calf serum in PBS solution containing 0,5% Brij);
- a washing buffer (for instance a PBS solution containing 0,5% Brij);
- a substrate (for instance a solution of 3,3',5,5'-tetramethylbenzidine in 0,05M citrate buffer);
- optionally a stop solution (for instance 0,3 M $H_2SO_4$).

**Electrophoresis**

One-dimensional SDS-PAGE was performed with precasted gel (Bio-Rad) with a 10% separation. All samples were prepared by dilution with an equal volume of Laemmli sample buffer (Bio-Rad, 1610737). In case of reducing conditions 2-mercaptoethanol is added to the Laemmli buffer and the sample is heated at 95°C for 5 minutes. Precision Plus Protein™ unstained standards (Bio-Rad) were used as molecular-weight markers. Gels were run in electrophoresis cell at 200 V until the dye front reached the bottom of the gel. The running buffer used was Tris/glycine/SDS (25 mM Tris, 192 mM glycine, 0.1% [w/v] SDS). Gels were stained with silver staining (Pierce Silver Stain Kit).

**Western blot**

The proteins separated on SDS-PAGE were transferred onto nitrocellulose (Whatman) by wet blotting for 1 hour at 100 V. The nitrocellulose membranes were blocked (5% skimmed milk in tris-buffered saline) for 3 hours, incubated overnight at 4°C with serum samples diluted in blocking buffer with 0,05% Tween-20, washed 3 times with tris-buffered saline with 0.05% Tween-20, incubated for 1 hour with either anti-IgG, anti-IgM or anti-IgA antibody conjugated with alkaline phosphatase in blocking buffer with 0,05% Tween-20, washed 3 times with tris-buffered saline with 0.05% Tween-20, incubated until detection with BCIP/NBT substrate solution, and stopped with water.

**Sensitivity and specificity**

The sensitivity is calculated as the % ratio between the number of true positive samples and the sum of the number of true positives plus the number of false negatives. The specificity is calculated as the % ratio between the number of true negative samples and the sum of the number of true negatives plus the number of false positives.

$$sensitivity = \frac{number\ of\ true\ positives}{number\ of\ true\ positives + number\ of\ false\ negatives}$$

$$specificity = \frac{number\ of\ true\ negatives}{number\ of\ true\ negatives + number\ of\ false\ positives}$$

**Examples**

**Example 1- Reactivity of the inactivated purified whole virus SARS-CoV-2**

The production process of the inactivated purified whole virus SARS-CoV-2 was optimized by setting up the conditions (e.g. β-propiolactone concentration between 0,05 and 0,5% [v/v], inactivation incubation times between 1 and 24 h, inactivation temperatures between 15°C and 37°C, centrifugation with/without sonication). The optimized process was verified by checking:

- the presence of the relevant proteins (i.e. spike glycoprotein, nucleocapsid protein, membrane protein, and envelope protein),

- the reactivity of the viral proteins in reducing and non-reducing conditions to reveal the role of conformation in antibody detection,
- the reactivity of the viral proteins in detecting antibodies in COVID-19 positive and negative samples.

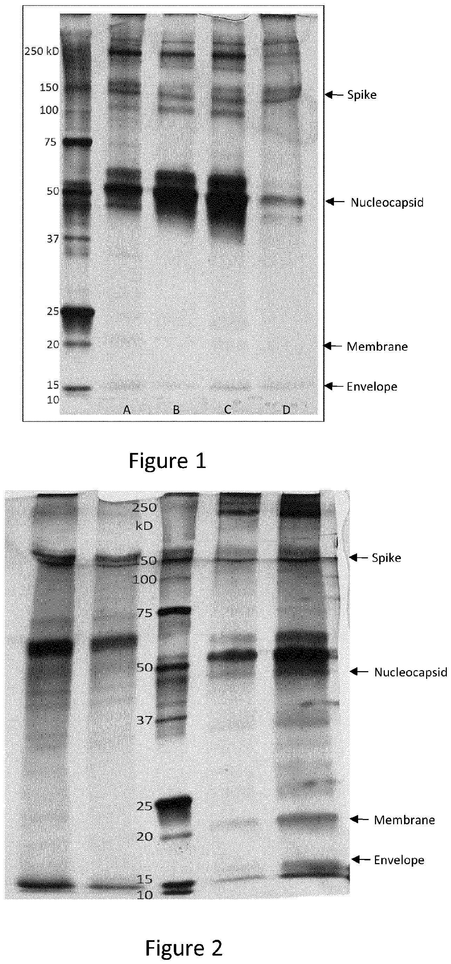

First, the efficacy and the reproducibility of the production process of the inactivated purified whole virus SARS-CoV-2 were evaluated by electrophoresis (Figure 1). Four different batches of production (i.e. lanes A, B, C, and D) were compared demonstrating the reproducibility of the method, which is reliable both in terms of total protein concentration and of protein reactivity. The electrophoresis demonstrates the presence of the four proteins: spike glycoprotein (i.e. amino acid mass $\approx$ 140 kDa), nucleocapsid protein (i.e. amino acid mass $\approx$ 46 kDa), membrane protein (i.e. amino acid mass $\approx$ 24 kDa), and envelope protein (i.e. amino acid mass $\approx$ 8 kDa). The molecular weight difference of the viral proteins between reducing and non-reducing conditions was evaluated by electrophoresis (Figure 2). The electrophoresis in non-reducing conditions allows the detection of the four viral proteins, on the contrary in reducing conditions not all the four viral proteins can be observed.

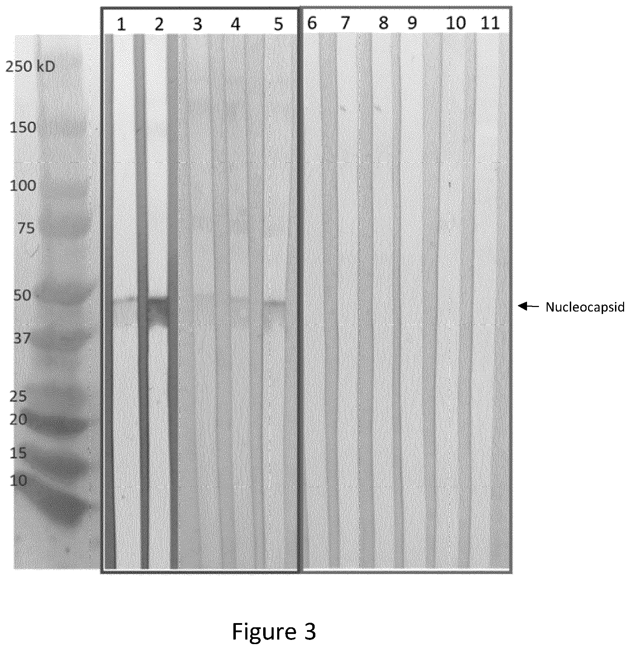

The reactivity of the viral proteins in detecting antibodies in COVID-19 positive and negative serum samples was evaluated by analyzing the inactivated purified whole virus SARS-CoV-2 in Western Blot (Figure 3). Results demonstrated that the negative serum samples do not contain any antibody specific for SARS-CoV-2. The positive samples demonstrate that, in the reducing conditions of the electrophoresis, only the nucleocapsid protein is recognized.

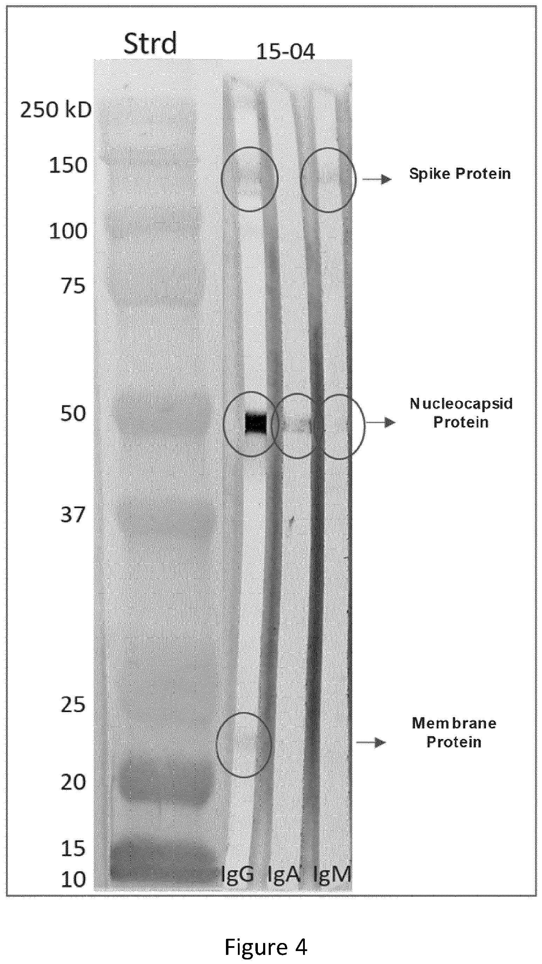

The reactivity of the viral proteins present in the inactivated purified whole virus SARS-CoV-2 was further analyzed by Western Blot in non-reducing conditions (Figure 4). Results demonstrate that in non-reducing conditions, in addition to the nucleocapsid protein, also the spike glycoprotein and the membrane protein are recognized by the IgG present in a COVID-19 positive serum sample from a patient declared recovered after 2 negative RT-PCR. IgM antibodies detect the nucleocapsid protein and the spike glycoprotein. IgA antibodies detect only the nucleocapsid protein.

These results demonstrate that:

- antibodies specific to the inactivated purified whole virus SARS-CoV-2 are present in serum samples after disease recovery and in patients but are not present in negatives;
- the 3 different types of antibodies IgG, IgM, and IgA can be detected in serum samples using the inactivated purified whole virus SARS-CoV-2;
- IgG, IgM, and IgA are directed against different viral proteins, thus supporting the hypothesis that the use of the inactivated purified whole virus SARS-CoV-2 instead of a single recombinant viral protein can provide a more sensitive diagnostic immunoassay;
- the different reactivity of IgG in reducing and non-reducing conditions may suggest that the reduction of the disulfide bonds of the viral proteins may alter their antigenicity.

**Example 2** - **Diagnostic performance of the ELISA test based on the inactivated purified whole virus SARS-CoV-2 for the detection of IgG antibodies**

According to the immunoenzymatic method of the invention, an ELISA test for the detection of IgG antibodies was developed. The detection was performed with an anti-human IgG monoclonal antibody labeled with horseradish peroxidase and reading the absorbance at 450 nm or 450/620 nm, using a specific substrate (3,3',5,5'-tetramethylbenzidine, Sigma, 860336) and $H_2SO_4$ (Sigma, 30743-M) 0,3 M as stop solution.

The diagnostic performances of the ELISA were evaluated in a clinical study comprising 495 serum samples characterized by an immunofluorescent test. Results of this comparison study are reported in Table 1 and demonstrate a strong correlation with immunofluorescence in terms of true positive (i.e. 62/67) and true negative samples (i.e. 410/428).

**Table 1.** Comparison study performed on a total of 495 serum samples tested with the ELISA of the present invention and an immunofluorescent test for the detection of anti-SARS-CoV-2 IgG.

| | | Reference (immunofluorescent test) | | |
| --- | --- | --- | --- | --- |
| | | Positive | Negative | Total |
| ELISA object of the invention | Positive | 62 | 18 | 80 |
| | Negative | 5 | 410 | 415 |
| | Total | 67 | 428 | 495 |

The diagnostic sensitivity and specificity of the anti-SARS-CoV-2 IgG ELISA of the present invention are reported in Table 2.

**Table 2.** Diagnostic sensitivity and specificity of the anti-SARS-CoV-2 IgG ELISA

| Diagnostic Sensitivity | 92.5% | CI95%: 83.6-96.8 |
|---|---|---|
| Diagnostic Specificity | 95.8% | CI95%: 93.4-97.3 |

**Example 3: Diagnostic performance of the ELISA test based on the inactivated purified whole virus SARS-CoV-2 for the detection of IgM antibodies**

According to the immunoenzymatic method of the invention, an ELISA test for the detection of IgM antibodies was developed. The detection was performed with an anti-human IgM monoclonal antibodies labeled with horseradish peroxidase and reading the absorbance at 450 nm or 450/620 nm, using a specific substrate (3,3',5,5'-tetramethylbenzidine) and $H_2SO_4$ 0,3 M as stop solution.

The diagnostic performances of the ELISA were evaluated in a clinical study comprising 397 serum samples characterized by an immunofluorescent test. Results of this comparison study are reported in Table 3 and demonstrate a strong correlation with immunofluorescence in terms of true positive (i.e. 57/65) and true negative samples (i.e. 322/332).

**Table 3.** Comparison study performed on a total of 397 serum samples tested with the ELISA of the present invention and an immunofluorescent test for the detection of anti-SARS-CoV-2 IgM.

| | | Reference (immunofluorescent test) | | |
|---|---|---|---|---|
| | | Positive | Negative | Total |
| ELISA | Positive | 57 | 10 | 67 |
| object of the invention | Negative | 8 | 322 | 330 |
| | Total | 65 | 332 | 397 |

The diagnostic sensitivity and specificity of the anti-SARS-CoV-2 IgM ELISA of the present invention are reported in Table 4.

**Table 4.** Diagnostic sensitivity and specificity of the anti-SARS-CoV-2 IgM ELISA

| Diagnostic Sensitivity | 87.7% | CI95%: 77.5-93.6 |
|---|---|---|
| Diagnostic Specificity | 97.0% | CI95%: 94.5-98.3 |

**Example 4: Diagnostic performance of the ELISA test based on the inactivated purified whole virus SARS-CoV-2 for the detection of IgA antibodies**

According to the immunoenzymatic method of the invention, an ELISA test for the detection of IgA antibodies was developed. The detection was performed with an anti-human IgA monoclonal antibodies labeled with peroxidase and reading the absorbance at 450 nm or 450/620 nm, using a specific substrate (3,3',5,5'-tetramethylbenzidine) and $H_2SO_4$ 0,3 M as stop solution.

The diagnostic performances of the ELISA were evaluated in a clinical study comprising 463 serum samples characterized by an immunofluorescent test. Results of this comparison study are reported in Table 5 and demonstrate a strong correlation with immunofluorescence in terms of true positive (i.e. 59/63) and true negative samples (i.e. 385/400).

**Table 5.** Comparison study performed on a total of 463 serum samples tested with the ELISA of the present invention and an immunofluorescent test for the detection of anti-SARS-CoV-2 IgA.

| | | Reference (immunofluorescent test) | | |
|---|---|---|---|---|
| | | Positive | Negative | Total |
| ELISA object of the invention | Positive | 59 | 15 | 74 |
| | Negative | 4 | 385 | 389 |
| | Total | 63 | 400 | 463 |

The diagnostic sensitivity and specificity of the anti-SARS-CoV-2 IgA ELISA of the present invention are reported in Table 6.

**Table 6.** Diagnostic sensitivity and specificity of the anti-SARS-CoV-2 IgA ELISA

| Diagnostic Sensitivity | 93.7% | CI95%: 84.7-97.5 |
| Diagnostic Specificity | 96.3% | CI95%: 93.9-97.7 |

In conclusion, we herein described a method for the production of an inactivated purified whole virus SARS-CoV-2 at large scale, which can be used for the preparation of immunoenzymatic assays for the detection of antibodies of class IgG, IgM, and IgA in serum samples.

The detected antibodies IgG, IgM, and IgA recognize different viral proteins, thus demonstrating the importance of a diagnostic immunoassay containing all the immunogenic proteins of the whole virus SARS-CoV-2. In fact, the use of the inactivated purified whole virus SARS-CoV-2 provides an antigenic combination of the recognized viral proteins. In addition, with this inactivation method the viral proteins, such as the spike glycoprotein, seem to maintain their antigenicity when the conformation is preserved by the presence of the disulfide bonds.

As reported in the state of the art, the detected anti-SARS-CoV-2 antibodies IgG, IgM, and IgA appear as a consequence of the different stages of the COVID-19 disease. Therefore, their detection is important for the diagnosis, follow-up, and identification of asymptomatic cases. The immunoenzymatic tests of the invention were tested on a significant number of confirmed COVID-19 patients and negative samples, in parallel with a reference immunofluorescent assay, demonstrating the high sensitivity and specificity of the method for the detection of the 3 classes of antibodies IgG, IgM, and IgA.

## References

[1] World Health Organization, Coronavirus disease 2019 (COVID-19), Situation Report - 91.

[2] World Health Organization, COVID-19 Strategy Update, April 14, 2020.

[3] World Health Organization, Laboratory testing for coronavirus disease (COVID-19) in suspected human cases: interim guidance, March 19, 2020.

[4] Anna Petherick. Developing antibody tests for SARS-CoV-2. Lancet. 2020, 395(10230), 1101-1102.

[5] Sandeep Kumar Vashist. In Vitro Diagnostic Assays for COVID-19: Recent Advances and Emerging Trends. Diagnostics. 2020, 10(4), 202.

[6] Wanbing Liu, Lei Liu, Guomei Kou, Yaqiong Zheng, Yinjuan Ding, Wenxu Ni, Qiongshu Wang, Li Tan, Wanlei Wu, Shi Tang, Zhou Xiong, Shangen Zheng. Evaluation of Nucleocapsid and Spike Protein-based ELISAs for detecting antibodies against SARS-CoV-2. Journal of Clinical Microbiology Mar 2020, JCM.00461-20; DOI: 10.1128/JCM.00461-20.

[7] Andrea Padoan, Laura Sciacovelli, Daniela Basso, Davide Negrini, Silvia Zuin, Chiara Cosma, Diego Faggian, Paolo Matricardi, Mario Plebani. Clinica Chimica Acta 2020; DOI: 10.1016/j.cca.2020.04.026.

[8] Renhong Yan, Yuanyuan Zhang, Yaning Li, Lu Xia, Yingying Guo, Qiang Zhou. Structural basis for the recognition of SARS-CoV-2 by full-length human ACE2. Science 2020, 367, 1444-1448.

[9] Alexandra C. Walls, Young-Jun Park, M. Alejandra Tortorici, Abigail Wall, Andrew T. McGuire, David Veesler. Cell 2020, 180, 1-12.

[10] Nicole C. Ammerman, Magda Beier-Sexton, Abdu F. Azad. Growth and Maintenance of Vero Cell Lines. Curr Protoc Microbiol. 2008 November; APPENDIX: Appendix-4E.

[11] Valeria Cagno. SARS-CoV-2 cellular tropism. Lancet Microbe 2020 April, DOI: 10.1016/S2666-5247(20)30008-2.

[12] Reed, L.J.; Muench, H. A simple method of estimating fifty percent endpoints. The American Journal of Hygiene. 1938, 27, 493-497.

## Claims

1. Method to inactivate SARS-CoV-2 whole virus particles from an isolated biological sample, comprising the steps of:

   a) inactivating SARS-CoV-2 whole virus particles by at least two additions of an inactivating reagent to said sample and optionally

    c) purifying inactivated SARS-CoV-2 whole virus particles.

2. The method of claim 1 wherein the additions of 0,1% (v/v) of β-propiolactone are performed at temperature ranging from 15°C to 37°C for at least 1 hour, preferably for 3 hours.

3. The method according to claim 1 or 2 wherein the SARS-CoV-2 whole virus particles belong to the strain 2019-nCoV/Italy-INMI1 (GenBank: SARS-CoV-2/INMI1-Isolate/2020/Italy: MT066156), or to a natural or recombinant derivative thereof.

4. A method of detecting one or more anti-SARS-CoV-2 virus specific antibody in a biological sample, the method comprising:

    a) incubating the biological sample with inactivated purified SARS-CoV-2 whole virus particles as prepared according to claim 1 to 3 under suitable conditions to obtain a SARS-CoV-2 virus/antibody complex;
    b) optionally washing to remove unbound material;
    c) detecting said SARS-CoV-2 virus /antibody complex.

5. The method of claim 4, wherein the anti-SARS-CoV-2 virus antibody is an IgG, IgM and/or an IgA antibody.

6. The method of claims 4 or 5 wherein said inactivated purified SARS-CoV-2 whole virus particles are pre-immobilized to a solid support.

7. The method of claim 6, wherein the solid support is a chip, column matrix material, a culture plate, a tube, a dish, a flask, a microtiter plate, a bead, microsphere, reactor vessel, wells, polystyrene or a combination thereof, preferably the solid support is a paramagnetic particle (PMP) or a latex magnetic particle (LMP).

8. The method of claims 4 to 7, wherein the detecting step is performed by means of a labelled secondary antibody, preferably a monoclonal antibody.

9. The method according to claim 8, wherein the secondary antibody is labeled with a radioactive isotope, or an enzyme, preferably the enzyme is peroxidase, alkaline phosphatase, β Galactosidase or acetylcholinesterase.

10. The method of claim 9, wherein the detecting step comprises measuring a signal from the label and comparing the signal to a control signal from a control biological sample known to be negative for SARS-CoV-2 virus antibodies.

11. The method of claims 4 to 10, wherein said inactivated purified SARS-CoV-2 whole virus particles comprise SARS-CoV-2 at least one of the following proteins :virus spike glycoprotein, nucleocapsid protein, membrane protein, an envelop protein or an immunogenic fragment thereof.

12. The method of claims 4 to 11 wherein the biological sample is blood, serum, plasma, body fluid, saliva and other secretions from the subject or tissue or cell extracts.

13. The method of claims 4 to 12 wherein the biological sample is obtained from the subject at least 3 days for IgM and IgA and at least 10 days for IgG after onset of symptoms of SARS-CoV-2 virus infection or is at risk of exposure to SARS-CoV-2 virus, or the subject has recovered from SARS-CoV-2 virus infection.

14. The method of claims 4 to 13, further comprising a quantification of amount of specific SARS-CoV-2 virus antibodies in the biological sample.

15. The method of claim 14, wherein the amount of the SARS-CoV-2 virus antibody in the biological sample is proportional to the amount of the complex detected.

16. A kit for working the method according to claims 4 to 15 comprising:

    a) a solid support coated with inactivated purified SARS-CoV-2 whole virus particles,
    b) at least one labeled secondary antibody able to bind to IgG or IgM or IgA,
    c) a negative control;

d) a positive control;
e) a cut-off control or calibrator;
f) a sample diluent;
g) a substrate solution;
h) a washing solution;
i) optionally a stop solution.

17. Use of inactivated purified SARS-CoV-2 whole virus particles obtained by the method according to claim 1 to 3 for detecting SARS-CoV-2 antibodies wherein said SARS-CoV-2 virus is strain 2019-nCoV/Italy-INMI1 (GenBank: SARS-CoV-2/INMI1-Isolate/2020/Italy: MT066156) or natural or recombinant derivative thereof.

Figure 1

Figure 2

Figure 3

Figure 4

**EP 3 913 369 A1**

# EUROPEAN SEARCH REPORT

Application Number

EP 20 17 5656

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WEILONG SHANG ET AL: "The outbreak of SARS-CoV-2 pneumonia calls for viral vaccines", NPJ VACCINES, vol. 5, no. 1, 6 March 2020 (2020-03-06), XP055727316, DOI: 10.1038/s41541-020-0170-0 * the whole document * | 1-17 | INV. G01N33/569 |
| Y | MA CUIQING ET AL: "From SARS-CoV to SARS-CoV-2: safety and broad-spectrum are important for coronavirus vaccine development", MICROBES AND INFECTION, ELSEVIER, PARIS, FR, vol. 22, no. 6, 11 May 2020 (2020-05-11), pages 245-253, XP086233893, ISSN: 1286-4579, DOI: 10.1016/J.MICINF.2020.05.004 [retrieved on 2020-05-11] * page 247 * | 1-17 | |
| Y | RAYMOND H SEE ET AL: "Comparative evaluation of two severe acute respiratory syndrome (SARS) vaccine candidates in mice challenged with SARS coronavirus", JOURNAL OF GENERAL VIROLOGY, SOCIETY FOR GENERAL MICROBIOLOGY, SPENCERS WOOD, GB, vol. 87, no. 3, 1 March 2006 (2006-03-01), pages 641-650, XP008138952, ISSN: 0022-1317, DOI: 10.1099/VIR.0.81579-0 * the whole document * | 1-17 | TECHNICAL FIELDS SEARCHED (IPC)  G01N |
| Y | CN 1 621 417 A (INST MICROBIOLOGY & EPIDEMIOLOGY ACADEMY MILITARY MEDICAL SCIENCES PLA) 1 June 2005 (2005-06-01) * page 5; example 1 * | 1-17 | |

-/--

The present search report has been drawn up for all claims

| Place of search | Date of completion of the search | Examiner |
|---|---|---|
| Munich | 9 October 2020 | Pinheiro Vieira, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 20 17 5656

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | M.R. CAPOBIANCHI ET AL: "Molecular characterization of SARS-CoV-2 from the first case of COVID-19 in Italy", CLINICAL MICROBIOLOGY AND INFECTION., vol. 26, no. 7, 27 March 2020 (2020-03-27), pages 954-956, XP055738054, United Kingdom, Switzerland ISSN: 1198-743X, DOI: 10.1016/j.cmi.2020.03.025 * the whole document * | 1-17 | |
| E | EP 3 715 847 A1 (EUROIMMUN MEDIZINISCHE LABORDIAGNOSTIKA AG [DE] ET AL.) 30 September 2020 (2020-09-30) * the whole document * | 1-17 | |

TECHNICAL FIELDS
SEARCHED (IPC)

The present search report has been drawn up for all claims

| Place of search | Date of completion of the search | Examiner |
|---|---|---|
| Munich | 9 October 2020 | Pinheiro Vieira, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 17 5656

This annex lists the patent family members relating to the patent documents cited in the above-mentioned European search report.
The members are as contained in the European Patent Office EDP file on
The European Patent Office is in no way liable for these particulars which are merely given for the purpose of information.

09-10-2020

| Patent document cited in search report | | Publication date | Patent family member(s) | Publication date |
|---|---|---|---|---|
| CN 1621417 | A | 01-06-2005 | NONE | |
| EP 3715847 | A1 | 30-09-2020 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *CHEMICAL ABSTRACTS*, 57-57-8
- *World Health Organization, Coronavirus disease 2019 (COVID-19), Situation Report - 91*
- *World Health Organization, COVID-19 Strategy Update,* 14 April 2020
- *World Health Organization, Laboratory testing for coronavirus disease (COVID-19) in suspected human cases: interim guidance,* 19 March 2020
- **ANNA PETHERICK.** Developing antibody tests for SARS-CoV-2. *Lancet,* 2020, vol. 395 (10230), 1101-1102
- Sandeep Kumar Vashist. In Vitro Diagnostic Assays for COVID-19: Recent Advances and Emerging Trends. *Diagnostics,* 2020, vol. 10 (4), 202
- **WANBING LIU ; LEI LIU ; GUOMEI KOU ; YAQIONG ZHENG ; YINJUAN DING ; WENXU NI ; QIONGSHU WANG ; LI TAN ; WANLEI WU ; SHI TANG.** Evaluation of Nucleocapsid and Spike Protein-based ELISAs for detecting antibodies against SARS-CoV-2. *Journal of Clinical Microbiology,* March 2020

- **ANDREA PADOAN ; LAURA SCIACOVELLI ; DANIELA BASSO ; DAVIDE NEGRINI ; SILVIA ZUIN ; CHIARA COSMA ; DIEGO FAGGIAN ; PAOLO MATRICARDI ; MARIO PLEBANI.** *Clinica Chimica Acta,* 2020
- **RENHONG YAN ; YUANYUAN ZHANG ; YANING LI ; LU XIA ; YINGYING GUO ; QIANG ZHOU.** Structural basis for the recognition of SARS-CoV-2 by full-length human ACE2. *Science,* 2020, vol. 367, 1444-1448
- **ALEXANDRA C. WALLS ; YOUNG-JUN PARK ; M. ALEJANDRA TORTORICI ; ABIGAIL WALL ; ANDREW T. MCGUIRE ; DAVID VEESLER.** *Cell,* 2020, vol. 180, 1-12
- **NICOLE C. AMMERMAN ; MAGDA BEIER-SEXTON ; ABDU F. AZAD.** Growth and Maintenance of Vero Cell Lines. *Curr Protoc Microbiol.,* November 2008
- **VALERIA CAGNO.** SARS-CoV-2 cellular tropism. *Lancet Microbe,* April 2020
- **REED, L.J. ; MUENCH, H.** A simple method of estimating fifty percent endpoints. *The American Journal of Hygiene,* 1938, vol. 27, 493-497